# EUROPEAN PATENT SPECIFICATION

(11) **EP 3 566 678 B1**
(45) Date of publication and mention of the grant of the patent: **05.07.2023**
(21) Application number: 18738680.0
(22) Date of filing: 03.01.2018
(51) Int. Cl.: A61F 2/32, A61F 2/36, A61F 2/30, A61F 2/34

(54) **ARTIFICIAL HIP JOINT COMPRISING COMPOSITE BALL**
KÜNSTLICHES HÜFTGELENK MIT VERBUNDKUGEL
ARTICULATION ARTIFICIELLE DE HANCHE COMPRENANT UNE BILLE COMPOSITE

(30) Priority: 10.01.2017 CN 201710016927
(43) Date of publication of application: 13.11.2019
(73) Proprietor: Tianjin Kingnow Technology Co., Ltd., West District TEDA Tianjin 300462 (CN)
(72) Inventor: Li, Guoan, Milton, MA 02186 (US); Li, Jianguo, Xihu District Nanchang Jiangxi 330009 (CN)
(74) Representative: Sun, Yiming
(86) International application number: PCT/CN2018/070086
(87) International publication number: WO 2018/130106

(56) References cited:
- WO-A1-96/04862
- CN-A- 102 824 232
- CN-A- 105 748 174
- CN-A- 106 580 521
- CN-U- 204 411 029
- DE-A1- 3 643 815
- DE-A1- 10 156 610
- DE-A1- 19 640 745
- GB-A- 1 340 436
- US-A1- 2004 059 429
- US-A1- 2005 102 035
- US-A1- 2007 032 877
- US-A1- 2013 190 889
- US-B1- 6 547 824
- US-B1- 6 610 095

## Description

### FIELD OF INVENTION

The present invention is defined in claim 1 as attached and relates to a field of medical instruments, and more particularly to an artificial hip joint with a composite ball head.

Specifically, the invention relates to an artificial hip joint with a composite ball head as defined in the generic part of claim 1 as attached.

### DESCRIPTION OF RELATED ARTS

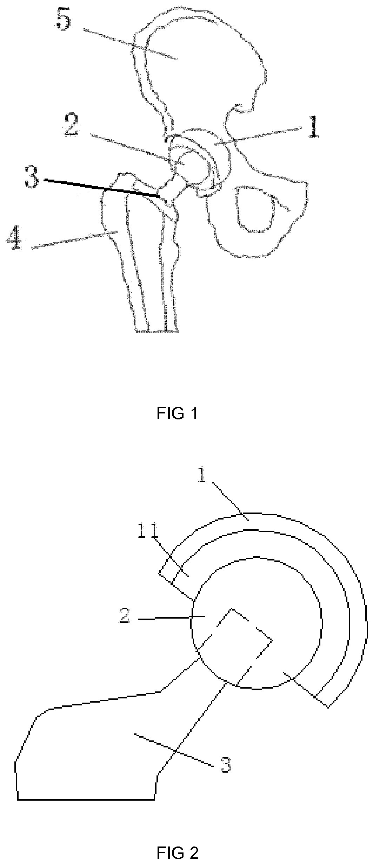

US 6,547,824 B1 discloses an artificial hip joint of the generic type as defined above. The human hip joint consists of acetabulum and femoral ball head. The ball head can rotate in the acetabulum to achieve normal movement of the hip joint. The human femoral ball head has a diameter of about 40 to 45 mm. When the hip joint is damaged, such as hip osteoarthritis, cartilage lesions or femoral neck rupture that causes the hip joint not functioning properly, hip joint replacement is required, that is, the artificial hip joint is placed through orthopedic surgery (see Fig. 1), which is the most commonly used surgical treatment method.

The conventional artificial hip joint is usually composed of a metal ball head, a metal artificial acetabulum and a non-metallic (usually high molecular weight polyethylene material) acetabular shell liner (see Fig. 2). Due to the limited strength of the polyethylene material, there is a certain requirement for the thickness of the liner. Thus, the thickness of the acetabular shell plus the thickness of the liner makes the inner diameter of the liner smaller than the inner diameter of human acetabulum. Accordingly, the diameter of the metal ball head is smaller than the diameter of the human femoral ball head. As a result, the contact area between the ball head and the liner is reduced, the contact stress is increased, and the wear of the liner is increased in practical use. At the same time, due to the reduction of the diameter of the ball head, the range of motion of the hip joint is reduced, affecting the normal life of the patient. Furthermore, it is easy to cause the femoral neck to collide with the liner boundary and cause the metal ball head to contact the liner boundary, resulting in problems such as high boundary stresses (edge loading) and damage to the liner material, and leading to failure of the artificial joint and thus revision surgery.

In order to solve these problems, ceramic materials have been introduced into artificial hip joints due to their high strength and wear resistance. However, due to the brittleness of the ceramic material, there is a certain requirement on the thickness of the liner, and the size of the ball head is still limited. The negative effects of the small ball head still exist, and the ceramics are prone to sharp noise due to friction, which is inconvenient for the patient's life and still needs to be greatly improved.

If the liner is not used and the metal ball head is used in combination with the metal shell, the metal ball head size can be made larger, making the large ball head possible. However, metal scraps and metal ions generated by metal friction are harmful to human body, so the design of metal ball head with metal shell has been abandoned by the clinic.

CN 105 748 174 A discloses a non-metallic hip joint prosthesis comprising an acetabular shell prosthesis, a non-metallic femoral head prosthesis and a femoral stem prosthesis, which reduces the thickness of the acetabular shell and allows the same-diameter acetabular shell to pair with a large-diameter ball head. However, the non-metallic femoral head prosthesis is difficult to achieve in practice, because sharp corners exist between the non-metallic femoral head prosthesis and the metal femoral stem that can lead to stress concentration in the non-metallic femoral head prosthesis and reduce its structural strength. It is difficult to carry the weight of the human body, and can be easily damaged once it is used in the artificial hip joint.

US 6,610,095 B1 discloses prosthetic joints, components for prosthetic joints, superhard bearing and articulation surfaces, diamond bearing and articulation surfaces, substrate surface topographical features, materials for making joints, bearing and articulation surfaces, and methods for manufacturing and finishing the same, including a prosthetic joint having substrate surface topographical features and at least one diamond articulation surface.

US 2004/0059429 A1 discloses a prosthetic orthopedic implant assembly including an implant body, and an elastomeric cover connected to the implant body by a mechanical fastener.

GB 1 340 436 A discloses a prosthetic device for the replacement of bone structure comprising a substrate with a pyrolytic carbon coating, which coating is biologically compatible with living tissue. The substrate may be of artificial graphite such as polycrystalline graphite, or boron carbide, silicon carbide, tantalum, molybdenum, tungsten, or mullite. In a related hip joint prosthesis, a ball section is fabricated from a polycrystalline carbon substrate with a pyrolytic carbon coating and is fastened to a metal strut.

US 6,547,824 B1 discloses a prosthetic joint having two load-bearing surfaces that are slidingly disposed relative to each other and define a region of frictional contact. The joint is thermally managed as heat is removed from the region during joint use by thermal conduction so that the surfaces are maintained approximately at or below specified temperatures. Heat flows preferentially through one member which includes one of the surfaces. High thermal conductivity materials are used in conjunction with various joint designs affording thermally managed devices.

Therefore, development of a clinically reliable large ball head artificial hip joint is still the objective of orthopedic bioengineering.

### SUMMARY OF THE PRESENT INVENTION

To overcome the deficiencies of the prior art, an objective of the present invention is to provide an artificial hip joint with a composite ball head, wherein no liner is used in the acetabulum so that the accommodating space of the acetabular shell is increased and the size of the composite ball head can be increased to increase the joint mobility and truly realize the advantages of the large ball head hip joint; at the same time, for the composite ball head, the metal ball head is wrapped with a non-metallic shell, which has large supporting area, reduces the contact stress of the material, has strong bearing capacity, is not easy to be damaged, effectively extends the service life of artificial joints, and enhances the usage of artificial joints.

Accordingly, in order to accomplish the above object, the present invention provides an artificial hip joint as defined by claim 1 as attached.

Preferred embodiments of the invention are defined by claims dependent upon claim 1 as attached.

The present invention adopts the metal material to make the artificial acetabulum, and the internal surface of the acetabulum is smooth, so that the composite ball head can freely rotate therein. Since no liner is used, the metal acetabulum is thin and the acetabular shell has a larger accommodation space. Since the thickness of the acetabulum can be made thinner, the acetabular shell has a larger accommodation space to accommodate the composite ball head. Therefore, the size of the composite ball head can be close to or exceed the actual size of the patient's femoral ball head according to the size of the acetabular shell, so as to achieve a true large ball head artificial hip joint.

Furthermore, the external surface of the metal artificial acetabulum may be coated with the coating that facilitates bone ingrowth.

The composite ball head of the present invention is formed by wrapping the external surface of the metal ball head with the non-metallic (high molecular weight polyethylene or PEEK or other wear-resistant non-metal) shell. The external surface of the metal ball head completely matches with the internal surface of the non-metallic shell without relative movement. The external surface of the non-metallic shell forms a pair of sliding surfaces with the smooth internal surface of the metal artificial acetabulum, and the composite ball head can freely rotate on the internal surface of the artificial acetabulum. The non-metallic shell made of wear-resistant non-metallic material avoids the brittleness of the ceramic material while has high strength, high toughness and excellent wear resistance. The metal ball is within the non-metallic shell, in such a manner that the composite ball head has an increased strength, a strong bearing capacity, and is not easy to be damaged.

One implementation manner of the present invention is that the metal ball head of the composite ball head may have the concave tapered space matching the top end of the femoral stem, and the metal ball head can be combined with the femoral stem through the concave tapered space and installed at the thigh bone. Such a connection method is stable and reliable in practical applications and is not easy to damage. The femoral stem can be any commercially available femoral stem, and the concave tapered space is formed to fit the top of the femoral stem. Therefore, the present invention can be combined with any conventional femoral stem and is easy to implement, providing a wide range of versatility.

Disclosed herein for reference only is an example, wherein the metal ball head can also be designed and manufactured integrally with the femoral stem, and is provided as a complete elongated metal femoral stem (including the metal ball head, the top end of the femoral stem and the femoral stem), which completely eliminates the contact between the metal ball head and the metal femoral stem. As a result, the artificial hip joint of the present invention would not introduce any metal-to-metal interface, and thereby would completely eliminate the origin of metal debris and metal ions.

The artificial hip joint with the composite ball head of the present invention does not use a liner in the acetabulum, and the accommodation space of the acetabular shell is increased, wherein the liner of the acetabular shell is made into the external shell of the metal ball head, and is directly combined with the metal ball head to form the composite ball head which is used in combination with the metal acetabulum shell. In this way, the size of the composite ball head can be increased to close to or exceed the size of the patient's femoral ball head, thereby increasing the joint mobility, reducing edge loading, reducing the collision between boundaries of the femoral stem and the acetabular shell, reducing the risk of dislocation of the acetabulum and the ball head, avoiding the negative effects of the small ball head, effectively prolonging the service life of the artificial joint, and enhancing the functional usage of the artificial joint. Compared with the conventional surgery, it is easy to implant without increasing any difficulty.

The present invention adopts the composite ball head, and the metal ball head is wrapped with the wear-resistant non-metal shell, wherein the support area is large, the contact stress is reduced, the bearing capacity is strong, stable and reliable. It is not easy to be damaged, and the service life of the artificial joint is prolonged.

The present invention can be used in conjunction with any conventional femoral stem, which is easy to implant, and has a wide range of versatility. The present invention can also completely eliminate the contact surface between the metal ball head and the metal femoral stem, and does not introduce any metal-to-metal interface, thereby completely eliminating the origin of metal debris and metal ions.

### BRIEF DESCRIPTION OF THE DRAWINGS

Fig. 1 is a sketch view of artificial hip joint replacement; wherein 1-artificial acetabulum, 2-artificial ball head, 3-femoral stem, 4-femur, 5-pelvis;
Fig. 2 is a sketch view of a conventional artificial hip joint; wherein 1-artificial acetabulum, 11-artificial acetabular shell liner, 2-artificial ball head, 3-femoral stem;
Fig. 3 is a sketch view of an artificial hip joint not according to the present invention; wherein 1-artificial acetabulum, 2-composite ball head, 21-non-metallic shell, 22-metal ball head, 3-femoral stem;
Fig. 4 is a structural view according to an example 1 not forming part of the present invention; wherein 1-artificial acetabulum, 21-non-metallic shell, 22-metal ball head, 23-concave tapered space, 3- femoral stem;
Fig. 5 is a structural view according to an example 2 not forming part of the present invention; wherein 1-artificial acetabulum, 21-non-metallic shell, 22-metal ball head, 3-femoral stem (for reference only);
Fig. 6 is a structural view according to an example 3 not forming part of the present invention; wherein 1-artificial acetabulum, 21-non-metallic shell, 22-metal ball head, 24-protruding member;
Fig. 7 is an exploded view according to an example 3 not forming part of the present invention; wherein 1-artificial acetabulum, 21-non-metallic shell, 22-metal ball head, 24-protruding member, 3-femoral stem;

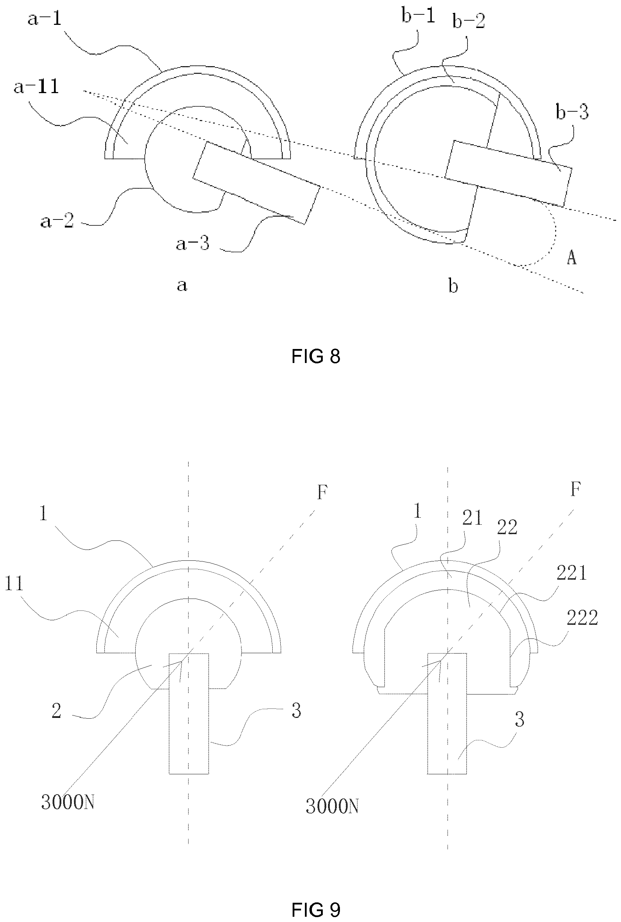

Fig. 8 illustrates comparison of active angles of an example not forming part the present invention and a conventional small spherical head hip joint; wherein a illustrates the active angle of the conventional small spherical head hip joint; wherein a-1: artificial acetabulum, a-11: artificial acetabular shell liner, a-2: artificial ball head, a-3: femoral stem; b illustrates the active angle of the exemplary artificial hip joint not forming part of the present invention; wherein b-1: artificial acetabulum, b-2: composite ball head, b-3: femoral stem; A: increment of active angle of the exemplary artificial hip joint;
Fig. 9 illustrates comparison of maximum stress of an example not forming part of the present invention and a conventional metal ball joint artificial hip joint, wherein a illustrates a conventional metal ball head hip joint, and b illustrates the exemplary artificial hip joint.

### DETAILED DESCRIPTION OF THE PREFERRED EMBODIMENT

The technical solutions in the embodiments of the present invention will be clearly and completely described below.

A conventional artificial hip joint usually comprises a metal artificial acetabulum 1 and an artificial ball head 2, wherein an internal surface of the artificial acetabulum 1 is provided with a non-metal (usually made of high molecular weight polyethylene material) acetabular shell liner 11, as shown in Fig. 2. Due to the limitations of the design, the acetabular shell liner 11 usually needs a certain thickness, so that the cavity formed on the internal surface of the acetabular shell liner 11 has a small volume, which limits the diameter of the metal ball head 2. Because the acetabular shell liner 11 and the artificial acetabulum 1 are relatively fixed to form an acetabular shell, and the metal ball head 2 is rotated as a movable member in the acetabular shell, the smaller internal surface of the acetabular shell and the small-sized metal ball head will bring the aforementioned all kinds of drawbacks.

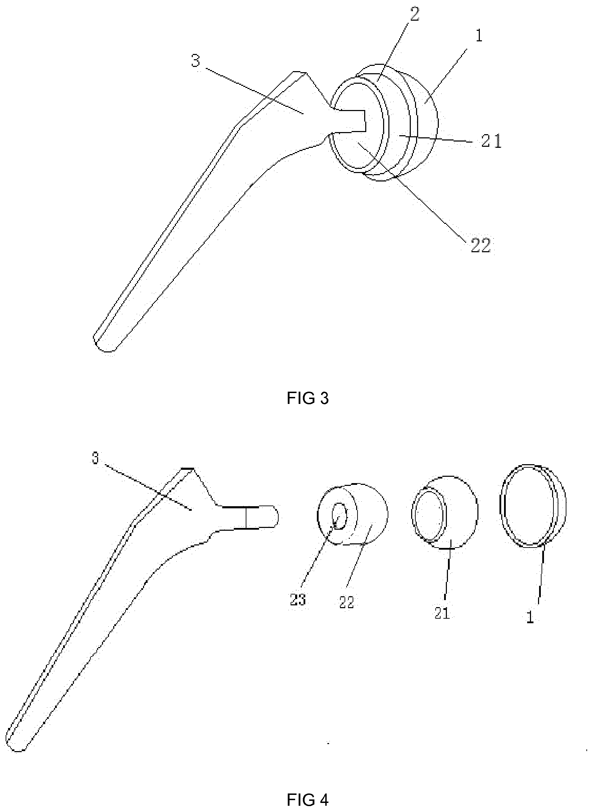

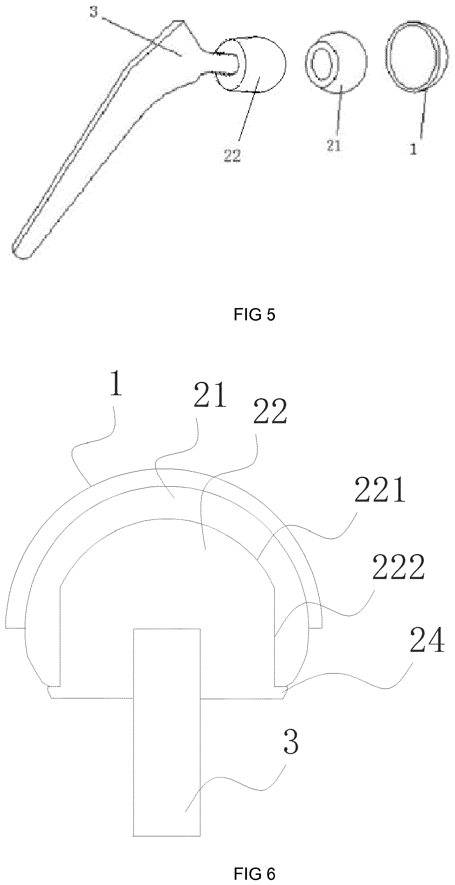

Therefore, the present invention provides an artificial hip joint with a composite ball head as shown in Fig. 6 further comprising a smooth curve connecting a spherical end portion 221 and a cylindrical protruding portion 222, comprising an artificial acetabulum 1 and the composite ball head 2 (see Fig. 3 for overall construct, however, metal ball head 2 of Fig. 3 is not according to the invention since it does not comprise the protruding member 24 described further below); wherein an internal surface of the artificial acetabulum 1 cooperates with an external surface of the composite ball head 2, and the composite ball head 2 can rotate within the artificial acetabulum 1; the internal surface of the artificial acetabulum 1 is directly in contact with the composite ball head 2 without a liner therebetween; the composite ball head 2 comprises a metal ball head 22 and a non-metallic shell 21 wrapped around an external surface of the metal ball head 22.

Since no liner is used in the present invention, the metal acetabulum 1 is thinner and the acetabular shell formed at the internal surface thereof has a larger accommodation space. Since the thickness of the artificial acetabulum 1 can be made thinner, the acetabular shell has a larger accommodation space to accommodate the composite ball head 2. Therefore, the size of the composite ball head 2 can be close to or exceed the actual size of the patient's femoral ball head according to the size of the acetabular shell, so as to achieve a true large ball head artificial hip joint.

Referring to Fig. 3, the femoral stem 3 can be separately produced or purchased as a prior art, which is then assembled with the metal ball head 22 of the present invention. Referring to Fig. 4, the metal ball head 21 can have a concave tapered space 23 which can cooperate with a top end of a femoral stem 3, and the metal ball head 21 can be combined with the femoral stem 3 through the concave tapered space 23, so as to be installed at a thigh bone.

Alternatively, the metal ball head of the composite ball head can be integrally made with a top end of a femoral stem. As a result, the composite ball head can be integrated with the femoral stem through the metal ball head as a single piece, which can reduce the interface of the metal parts, thereby greatly reducing the risk of wear and metal powder due to contact between the metal parts.

The artificial acetabulum 1 is made of a metallic material. Some metal materials have high strength, toughness and wear resistance, which can make the artificial acetabulum 1 thin enough to obtain a larger acetabular shell, thus bringing the possibility of using a large-sized composite ball head 2.

In practical applications, the non-metallic shell 21 of the composite ball head 2 is made of a wear resistant non-metallic material.

In practical applications, the non-metallic shell of the composite ball head is made of a high molecular weight polyethylene material or a PEEK (polyether ether ketone) material.

In practical applications, the internal surface of the artificial acetabulum (namely the acetabular shell surface) is a smooth surface.

Preferably, an external surface of the artificial acetabulum 1 is coated with a coating which facilitates bone ingrowth. The external surface refers to a convex spherical surface of the artificial acetabulum.

Preferably, a size of the composite ball head is close to, identical to, or exceeds a size of a patient femoral head according to a size of the artificial acetabulum.

The present invention adopts the metal material to make the artificial acetabulum, and the internal surface of the acetabulum is smooth, so that the composite ball head can freely rotate therein. Since no liner is used, the metal acetabulum is thin and the acetabular shell has a large accommodation space. Since the thickness of the acetabulum can be made thinner, the acetabular shell has a larger accommodation space to accommodate the composite ball head. Therefore, the size of the composite ball head can be close to or exceed the actual size of the patient's femoral ball head according to the size of the acetabular shell, so as to achieve a true large ball head artificial hip joint.

The composite ball head of the present invention is formed by wrapping the external surface of the metal ball head with the non-metallic (high molecular weight polyethylene or PEEK or other wear-resistant non-metal) shell. The external surface of the metal ball head completely matches with the internal surface of the non-metallic shell without relative movement. The external surface of the non-metallic shell forms a pair of sliding surfaces with the smooth internal surface of the metal artificial acetabulum, and the composite ball head can freely rotate on the internal surface of the artificial acetabulum. The non-metallic shell made of wear-resistant non-metallic material avoids the brittleness of the ceramic material while has high strength, high toughness and excellent wear resistance. The metal ball is within the non-metallic shell, in such a manner that the composite ball head has an increased strength, a strong bearing capacity, and is not easy to be damaged.

One implementation manner of the present invention is that the metal ball head of the composite ball head may have a concave tapered space matching the top end of the femoral stem, and the metal ball head can be combined with the femoral stem through the concave tapered space and install at the thigh bone. Such a connection method is stable and reliable in practical applications and is not easily damaged. The femoral stem can be any commercially available femoral stem, and the concave tapered space is formed to fit the top of the femoral stem. Therefore, the present invention can be combined with any conventional femoral stem and is easy to implement, providing a wide range of versatility.

Also disclosed herein for reference only is that the metal ball head can also be designed and manufactured integrally with the femoral stem, and is provided as a complete elongated metal femoral stem (including the metal ball head, the top end of the femoral stem and the femoral stem), which completely eliminates the contact between the metal ball head and the metal femoral stem. As a result, the artificial hip joint of the present invention does not introduce any metal-to-metal interface, and thereby completely eliminates the origin of metal debris and metal ions.

The artificial hip joint with the composite ball head of the present invention does not use liner in the acetabulum, and the accommodation space of the acetabular shell is increased, wherein the liner of the acetabular shell is made into the shell of the metal ball head, and is directly combined with the metal ball head to form the composite ball head which is used in combination with the metal shell. In this way, the size of the composite ball head can be increased to close to or exceed the size of the patient's femoral ball head, thereby increasing the joint mobility, reducing the boundary stress/edge loading, reducing the collision between boundaries of the femoral stem and the acetabular shell, reducing the risk of dislocation of the acetabulum and the ball head, avoiding the negative effects of the small ball head, effectively prolonging the service life of the artificial joint, and enhancing the functional usage of the artificial joint. Compared with the conventional surgery, it is easy to implant without increasing any difficulty.

The present invention adopts the composite ball head, and the metal ball head is wrapped with the wear-resistant non-metal shell, wherein the support area is large, the contact stress is reduced, the bearing capacity is strong, stable and reliable. It is not easy to be damaged, and the service life of the artificial joint is prolonged.

The present invention can be used in conjunction with any conventional femoral stem, which is easy to implement, and has a wide range of versatility. The present invention can also completely eliminate the contact surface between the metal ball head and the metal femoral stem, and does not introduce any metal-to-metal interface, thereby completely eliminating the origin of metal debris and metal ions.

Referring to Fig. 4 showing an example not forming part of the invention, the composite ball head 2 comprises the non-metallic shell 21 and the metal ball head 22, wherein an end face of the metal ball head 22 that is not in contact with the non-metallic shell can be provided with concave tapered space 23 that can cooperate with a top end of the femoral stem 3. The top end of the femoral stem 3 can be

inserted into the concave tapered space 23 to join the femoral stem 3 to the composite ball head 2. The femoral stem 3 can be any femoral stem available on the market. When in use, the metal artificial acetabulum can be mounted to the corresponding position of the pelvic acetabulum of the patient by squeezing/press fitting or cementing, and the composite ball head can be attached to the thigh bone through the concave tapered space of the metal ball head and the femoral stem. Such a connection method is stable and reliable in practical applications and is not easy to be damaged.

The femoral stem can be any commercially available femoral stem, and the concave tapered space can be formed to match the top of the femoral stem. Therefore, the present invention can be combined with any conventional femoral stem and is easy to implement, providing a wide range of versatility.

According to the present invention, the artificial hip joint with the composite ball head comprises an artificial acetabulum 1 and a composite ball head 2, wherein the artificial acetabulum 1 is made of a metal material, and the internal surface thereof is a surface without a liner, which may be a smooth slip surface.

The external surface of the artificial acetabulum 1 may be coated to facilitate ingrowth of the bone after implantation. The composite ball head 2 comprises a metal ball head 22 made of a metal material and a non-metallic shell 21 wrapped around the metal ball head. The non-metallic shell can be made of high molecular weight polyethylene or PEEK (polyether ether ketone) material, and the external surface of the metal ball head is tightly wrapped.

Referring to Fig. 5 disclosed herein for reference only, the composite ball head 2 comprises the non-metallic shell 21 and the metal ball head 22. The metal ball head 22 can be

integrally formed with the top end of the femoral stem 3 to form a fully extended metal stem, thereby combining the femoral stem, the metal ball head, and the non-metallic shell. In use, the metal artificial acetabulum can be attached to the pelvic acetabulum of the patient by squeezing/press fitting or bone cement, and the composite ball head is attached to the thigh bone through a femoral stem integrated with the metal ball head.

In this example, since the metal ball head and the top end of the femoral stem can be

integrally arranged, the metal-to-metal interface in the ball head can be completely eliminated, and would thereby completely eliminate metal debris and metal ions generated by the metal-to-metal interface of.

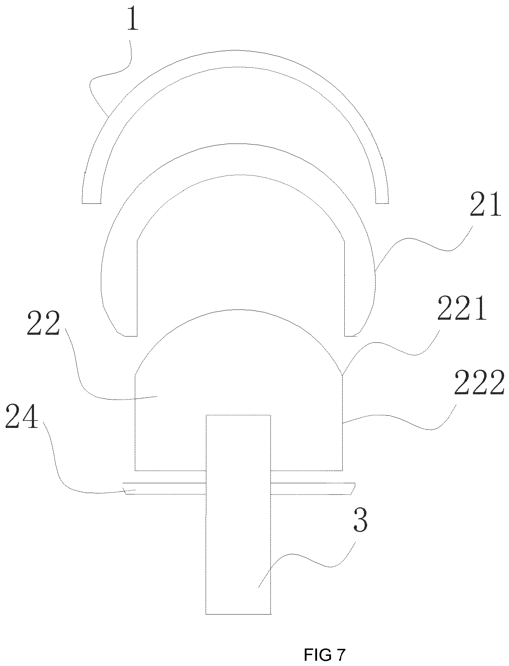

According to the present invention, a protruding member 24 that is in contact with the external bottom of the non-metallic shell 21 is provided in the bottom portion of the metal ball head 22, as shown in Figs. 6 and 7. The protruding member 24 can enhance the stability of the non-metallic shell 21.

Referring to Figs. 6 and 7, the metal ball head 22 comprises a spherical end portion 221 and a cylindrical protruding portion 222, wherein a cavity formed by the internal surface of the non-metallic shell 21 matches with an external shape of the metal ball head 22. With such structure, the connection tightness between the metal ball head 22 and the non-metallic shell 21 can be greatly improved, the slip between the two can be effectively prevented, and the stability of the product can be improved. A smooth curve (not shown) is used between the spherical end surface 221 and the cylindrical protruding portion 222. In some embodiments, the cross-sectional dimension of the cylindrical protruding portion 222 can gradually decrease from the side near the spherical end surface 221 toward the side of the femoral stem 3, or can gradually decrease in an undulating shape, which can further improve the stability of the product.

Since the present invention has no liner, the acetabulum can be thin, the diameter of the composite ball head can be larger, the contact area between the ball head and the acetabulum can be increased, the contact stress is reduced, and the active angle of the ball head in the acetabular shell can be large, which can increase the range of motion of the hip joint.

Fig. 8 illustrates comparison of active angles of an example not forming part of the present invention since it lacks the cylindrical protruding portion 222, and a conventional small spherical head hip joint. Although Fig. 8 b is not representative of the present invention, the comparison of the active angles also applies to the composite ball head comprising the protruding member 24 and the cylindrical protruding portion 222 according to the present invention. Fig. 8 a illustrates the active angle of the conventional small spherical head hip joint. The artificial acetabulum a-1 is provided with a liner a-11, the thickness of the acetabulum plus the thickness of the liner makes the inner diameter of the liner smaller than the inner diameter of human acetabulum. Correspondingly, the diameter of the metal ball head a-2 is reduced, causing the active angle of the metal ball head to decrease, which easily causes the collision between boundaries of the femoral stem a-3 and the liner a-11, as well as between boundaries of the metal ball head and the liner 11. Correspondingly, the range of motion of the hip joint is reduced. At the same time, since the diameter of the ball head is reduced, the contact area between the ball head and the liner is small, and the contact stress is increased. In actual use, liner wear is likely to be increased, and the service life of the joint is reduced. b of Fig. 8 illustrates the active angle of the example of the artificial hip joint not forming part of the present invention since it lacks the cylindrical protruding portion 222 and the protruding member 24; wherein the metal acetabulum b-1 is not provided with the liner, so the metal acetabulum is thinner and the acetabular shell has a larger accommodation space. Therefore, the composite ball head b-2 can be used and the size of the composite ball head can be close to or exceed the actual size of the patient's femoral ball head according to the size of the acetabular shell, thereby increasing the active angle and the range of motion of the hip joint. The angle A is increment of active angle of the exemplary artificial hip joint with the conventional small spherical head hip joint.

Due to the increased active angle and the range of motion of the exemplary artificial hip joint, the present invention overcomes the problems of small ball head hip joint in which the femoral neck collides with the liner boundary and the metal ball head contacts with the liner boundary, limiting the use of the hip joint and the realization of physiological functions. As a result, the service life of the artificial joint of the present invention will naturally increase. At the same time, the present invention adopts a composite ball head, and the metal ball head is wrapped with non-metal shell which may be wear-resistant, wherein the support area is large, the contact stress is reduced, the bearing capacity is strong, which is stable and reliable, and is not easy to be damaged.

Fig. 9 illustrates comparison of maximum stress of an example not forming part of the present invention, since it lacks the smooth transition between the spherical end portion 221 and the cylindrical protruding portion 222, and a conventional metal ball joint artificial hip joint, wherein a illustrates a conventional metal ball head hip joint, and b illustrates the exemplary artificial hip joint. F in Fig. 9 indicates the load direction at a certain angle. Table 1 shows comparison of the maximum stress of the exemplary artificial hip joint and the conventional metal ball head hip joint under the same load conditions.

**Table 1: comparison of the maximum stress in the polyethylene component of the exemplary artificial hip joint and the conventional metal ball head**

| Load direction | Peak von Mises Stress (MPa) | | |
|---|---|---|---|
| | Prior art | Composite ball head | Stress decrease |
| 0° | 14.82 | 12 | 19.03% |
| 45° | 14.51 | 12.01 | 17.23% |
| 70° | 17.35 | 15.02 | 13.43% |

It can be seen from Table 1 that under the same load, the maximum stress generated by the composite ball head of the exemplary artificial hip joint is lower than the maximum stress of the conventional metal ball head hip joint.

The present invention provides the artificial hip joint with the composite ball head, which maintains all advantages of large ball head hip joints, such as increasing the joint mobility, reducing the boundary stress/edging loading, reducing the collision between boundaries of the femoral stem and the acetabular shell, reducing the risk of dislocation of the acetabulum and the ball head, avoiding the negative effect of the small ball head, effectively prolonging the service life of the artificial joint, and enhancing the useful function of the artificial joint. Compared with the conventional surgery, it is easy to implant without increasing any difficulty. The present invention adopts the composite ball head, and the metal ball head is wrapped with the non-metal shell, which may be wear-resistant, wherein the support area is large, the contact stress is reduced, the bearing capacity is strong, stable and reliable. It is not easy to damage, and the service life of the artificial joint is prolonged.

The present invention can be used in conjunction with any conventional femoral stem, which is easy to implement, and has a wide range of versatility. The present invention can also completely eliminate the contact surface between the metal ball head and the metal femoral stem, and does not introduce any metal-to-metal interface, thereby completely eliminating the origin of metal debris and metal ions.

## Claims

1. An artificial hip joint with a composite ball head (2), comprising an artificial acetabulum (1) and the composite ball head; wherein: the artificial acetabulum is made of a metal material; an internal surface of the artificial acetabulum cooperates with an external surface of the composite ball head, and the composite ball head can rotate within the artificial acetabulum; the composite ball head comprises a metal ball head (22) and a non-metallic shell (21) disposed around an external surface of the metal ball head; wherein the metal ball head comprises a spherical end portion (221) and a protruding member (24), and the protruding member is in contact with an external bottom end of the non-metallic shell, wherein the non-metallic shell is separate from the metal ball head and is wrapped around the external surface; wherein a cavity formed by an internal surface of the non-metallic shell matches with an external shape of the metal ball head; the artificial hip joint is configured such that in use the internal surface of the artificial acetabulum is directly in contact with the composite ball head without a liner therebetween; **characterized in that**
the metal ball head comprises a cylindrical protruding portion (222) connected to the spherical end portion by a smooth curve.

2. The artificial hip joint, as recited in claim 1, wherein the metal ball head has a concave tapered space which cooperates with a top end of a femoral stem (3), and the metal ball head is combined with the femoral stem through the concave tapered space, so as to be installed at a thigh bone.

3. The artificial hip joint, as recited in claim 1, wherein the metal ball head is integrally provided with a femoral stem (3).

4. The artificial hip joint, as recited in claim 1, wherein the non-metallic shell of the composite ball head is made of a wear resistant non-metallic material.

5. The artificial hip joint, as recited in claim 4, wherein the non-metallic shell of the composite ball head is made of a high molecular weight polyethylene material or a PEEK (polyether ether ketone) material.

6. The artificial hip joint, as recited in claim 1, wherein an external surface of the artificial acetabulum is coated with a coating which facilitates bone ingrowth.

## Patentansprüche

1. Künstliches Hüftgelenk mit einem Verbundkugelkopf (2), umfassend eine künstliche Hüftgelenkpfanne (1) und den Verbundkugelkopf; wobei: die künstliche Hüftgelenkpfanne aus einem Metallmaterial hergestellt ist; eine Innenfläche der künstlichen Hüftgelenkpfanne mit einer Außenfläche des Verbundkugelkopfs zusammenwirkt und der Verbundkugelkopf in der künstlichen Hüftgelenkpfanne drehen kann; der Verbundkugelkopf einen Metallkugelkopf (22) und eine nicht metallische Schale (21) umfasst, die um eine Außenfläche des Metallkugelkopfs angeordnet ist; wobei der Metallkugelkopf einen sphärischen Endabschnitt (221) und ein vorstehendes Element (24) umfasst und das vorstehende Element mit einem äußeren unteren Ende der nicht metallischen Schale in Kontakt ist, wobei die nicht metallische Schale von dem Metallkugelkopf getrennt ist und um die Außenfläche geschlungen ist, wobei ein Hohlraum, der durch eine Innenfläche der nicht metallischen Schale gebildet wird, mit einer Außenfläche des Metallkugelkopfs übereinstimmt; das künstliche Hüftgelenk so ausgelegt ist, dass in Verwendung die Innenfläche der künstlichen Hüftgelenkpfanne in direktem Kontakt mit dem Verbundkugelkopf steht, ohne dazwischen liegende Auskleidung; **dadurch gekennzeichnet, dass**
der Metallkugelkopf einen zylindrischen vorstehenden Abschnitt (222) umfasst, der mit dem sphärischen Endabschnitt durch eine sanfte Kurve verbunden ist.

2. Künstliches Hüftgelenk nach Anspruch 1, wobei der Metallkugelkopf einen konkaven verjüngten Raum aufweist, der mit einem oberen Ende eines Femurschafts (3) zusammenwirkt, und der Metallkugelkopf mit dem Femurschaft durch den konkaven verjüngten Raum kombiniert ist, um so bei einem Oberschenkelknochen installiert zu werden.

3. Künstliches Hüftgelenk nach Anspruch 1, wobei der Metallkugelkopf einstückig mit einem Femurschaft (3) bereitgestellt ist.

4. Künstliches Hüftgelenk nach Anspruch 1, wobei die nicht metallische Schale des Verbundkugelkopfs aus einem abriebbeständigen nicht metallischen Material hergestellt ist.

5. Künstliches Hüftgelenk nach Anspruch 4, wobei die nicht metallische Schale des Verbundkugelkopfs aus einem Polyethylenmaterial hohen Molekulargewichts oder einem PEEK- (Polyetheretherketon) Material hergestellt ist.

6. Künstliches Hüftgelenk nach Anspruch 1, wobei eine Außenfläche der künstlichen Hüftgelenkpfanne mit einer Beschichtung beschichtet ist, die Knocheneinwachsen erleichtert.

## Revendications

1. Articulation de hanche artificielle comportant une rotule composite (2) comprenant un acétabulum (1) et la rotule composite, dans laquelle : l'acétabulum artificiel est composé d'un matériau métallique ; une surface interne de l'acétabulum artificiel coopère avec une surface externe de la rotule composite, et la rotule composite peut tourner dans l'acétabulum artificiel ; la rotule composite comprend une rotule métallique (22) et une coque non métallique (21) disposée autour d'une surface externe de la rotule métallique ; la rotule métallique comprend une section terminale sphérique (221) et un élément proéminent (24), et l'élément proéminent et en contact avec une extrémité inférieure externe de la coque non métallique, la coque non métallique étant séparée de la rotule métallique et entourant la surface externe ; une cavité formée par une surface interne de la coque non métallique correspondant à une forme externe de la rotule métallique ; l'articulation de hanche artificielle étant configurée de manière à ce que, à l'usage, la surface interne de l'acétabulum artificiel soit directement en contact avec la rotule composite sans doublure entre eux ; **caractérisée en ce que**
la rotule métallique comprend une section proéminente cylindrique (222) connectée à la section terminale sphérique par une courbe modérée.

2. Articulation de hanche artificielle selon la revendication 1, dans laquelle la rotule métallique comporte un espace fuselé concave qui coopère avec une extrémité supérieure d'une tige fémorale (3), et la rotule métallique est combinée à la tige fémorale par l'espace fuselé concave de manière à être installé sur un os de cuisse.

3. Articulation de hanche artificielle selon la revendication 1, dans laquelle la rotule métallique est intégralement pourvu e d'une tige fémorale (3).

4. Articulation de hanche artificielle selon la revendication 1, dans laquelle la coque non métallique de la rotule composite est composée d'un matériau non métallique résistant à l'usure.

5. Articulation de hanche artificielle selon la revendication 4, dans laquelle la coque non métallique de la rotule composite est composée d'un matériau à base de polyéthylène à poids moléculaire élevé ou d'un matériau à base de polyéther éther cétone (PEEK)).

6. Articulation de hanche artificielle selon la revendication 1, dans laquelle une surface externe de l'acétabulum artificiel est revêtue avec un revêtement qui facilite la croissance interne de l'os.
